Europäisches Patentamt

European Patent Office

Office européen des brevets

(11) Veröffentlichungsnummer: **0 324 162 B1**

# EUROPÄISCHE PATENTSCHRIFT

(45) Veröffentlichungstag der Patentschrift: **09.03.94**

(51) Int. Cl.5: **C12N 15/62**, C12P 21/00

(21) Anmeldenummer: **88121675.8**

(22) Anmeldetag: **24.12.88**

(54) **Verfahren zur gentechnischen Herstellung von Antikörpern.**

(30) Priorität: **31.12.87 DE 3744595**

(43) Veröffentlichungstag der Anmeldung:
**19.07.89 Patentblatt 89/29**

(45) Bekanntmachung des Hinweises auf die
Patenterteilung:
**09.03.94 Patentblatt 94/10**

(84) Benannte Vertragsstaaten:
**AT BE CH DE ES FR GB GR IT LI LU NL SE**

(56) Entgegenhaltungen:
**EP-A- 0 120 694**
**EP-A- 0 196 864**
**EP-A- 0 234 592**

**CHEMICAL ABSTRACTS**

**Gene, 1984, 27 (3), S. 315-322**

**PROC. NATL. ACAD. SCI. USA Band 81, Juni 1984, S. 3273-3277, Washington, US; S. CABILLY et al.: "Generation of antibody activity from immunoglobulin polypeptide chains produced in Escherichia coli"**

(73) Patentinhaber: **Plückthun, Andreas, Dr.**
**Jägerwirtstrasse 3**
**D-81373 München(DE)**

Patentinhaber: **Skerra, Arne**
**Cheruskerweg 6**
**D-65187 Wiesbaden(DE)**

(72) Erfinder: **Plückthun, Andreas, Dr.**
**Jägerwirtstrasse 3**
**D-81373 München(DE)**
Erfinder: **Skerra, Arne**
**Cheruskerweg 6**
**D-65187 Wiesbaden(DE)**

(74) Vertreter: **Fischer, Hans-Jürgen, Dr. et al**
**Hoechst AG,**
**Zentrale Patentabteilung,**
**Gebäude F 821**
**D-65926 Frankturt am Main (DE)**

CHEMICAL ABSTRACTS, Band 109, Nr. 3, 18. Juli 1988, Zusammenfassung Nr. 21334w; MARC BETTER et al.: "Escherichia coly secretion of an active chimeric antibody fragment"; & SCIENCE 1988, Band 240, Nr. 4855, S. 1041-1043

BIOSIS DATABASE, Zusammenfassung Nr. 85099940; K. KITAI et al.: "Extracellular production of human immunoglobulin G FC region H-IGG-FC by escherichia-coli"; &APPL. MICROBIOL. BIOTECHNOL., Band 28, Nr. 1, 1988, S. 52- 56

PATENT ABSTRACTS OF JAPAN, Band 12, Nr. 56 (C-477), 19. Februar 1988; & JP-A-62 201 581 (RIKAGAKU KENKYUSHO) 5. Sept. 1987

CHEMICAL ABSTRACTS, Band 101, Nr. 9, 27. Aug. 1984, Zusammenfassung Nr. 67017h; ORNA ZEMEL-DREASEN et al.: "Secretion and processing of an immuno- globulin light chain in Escherichia coli"; & GENE, 1984, Band 27, Nr. 3, S. 315-322

2

# EP 0 324 162 B1

**Beschreibung**

Die Expression von Antikörpern in Hefe wurde beschrieben (C.R. Wood, M.A. Boss, J.H. Kenten, J.E. Calvert, N.A. Roberts and J.S. Emtage, Nature 314, 446 (1985)), aber nur ein sehr kleiner Anteil des exprimierten Proteins erwies sich als funktionell. In E. coli konnten bisher Antikörper-Proteine nur in denaturierter Form erhalten werden (M.A. Boss, J.H. Kenten, C.R. Wood and J.S. Emtage, Nucleic Acids Res. 12, 3791 (1984); S. Cabilly, A.D. Riggs, H. Pande, J.E. Shively, W. E. Holmes, M. Rey, L. J. Perry R. Wetzel and H.L; Heyneker, Proc. Natl. Acad. Sci. U.S.A. 81, 3273 (1984)). Gene, 1984, 27, S.315-322 beschreibt die Expression und Sekretion einer leichten Kappa-Kette (L-321) einer Haus Myelomazelle in E. coli: Die Reinigung aktiver Antikörper oder Antikörperfragmente aus Hefe oder anderen Mikroorganismen ist noch nicht bekannt. Proteinfaltungsversuche haben bisher nur zu einem geringen Prozentsatz richtig gefalteter rekombinanter Antikörper Proteine geführt. Außerdem ist es schwierig, die gewünschten funktionellen Proteine von unerwünschten und nicht funktionellen Proteinen abzutrennen, was die genaue Messung von Bindungskonstanten, Ausbeuten bei der Faltung, spektralen Eigenschaften und dergleichen und die Anwendung in Therapie und Technik erschwert. Die Optimierung der Faltungsbedingungen ist deshalb außerordentlich schwierig, zumal es keine bewährten Verfahren und Meßmethoden für die Rückfaltung gibt.

Die Expression funktionaler kompletter Antikörper oder funktioneller Bindungsdomänen von Antikörpern in bakteriellen Expressionssystemen ist bisher nicht bekannt und die Aussichten zu ihrer Verwirklichung wurden pessimistisch beurteilt (S.L. Morrison, Science 229, 1202 (1985), M.A. Boss and C.R. Wood, Immunol. Today 6, 12 (1985)).

Ein solches System wäre sehr wünschenswert, da die gentechnischen Verfahren insbesondere für E. coli gut ausgearbeitet sind und aufgrund des schnellen Wachstums die Massenproduktion erleichtert ist, was ökonomisch von erheblicher Bedeutung ist.

Die Erfindung bezieht sich deshalb auf die Herstellung von funktionsfähigen Antikörpen, deren funktionelle Fragmente oder von funktionellen Fusionsproteinen aus Antikörperdomänen und anderen Proteinen in Bakterien, vorzugsweise Gram-negativen Bakterien, insbesondere in E. coli. Das erfindungsgemäße Verfahren ist dadurch gekennzeichnet, daß man die Gene für die Schwere und leichte Kette des Antikörpermoleküls oder -fragments je an eine Signalsequenz koppelt, die den Transport der Polypeptidketten durch die cytoplasmatische Membran bewirkt und abgespalten werden kann, die Genstrukturen zur Expression bringt und das funktionelle Protein aus dem periplasmatischen Raum oder dem Medium isoliert. Bevorzugte Ausgestaltungen dieser Erfindung werden im folgenden näher erläutert bzw. in den Patentansprüchen definiert.

Die Koppelung der Gene für die einzelnen mit Signal-Sequenzen versehen Ketten erfolgt vorteilhaft in der Art eines regulierbaren Operon-Systems, das die gleichzeitige Expression durch eine gemeinsame Steuerungsregion bewirkt. Auf diese Weise werden die einzelnen Proteinketten im angenähert stöchiometrischen Verhältnis gemeinsam exprimiert und in den periplasmatischen Raum transportiert, wo die Verbindung zum funktionellen Molekül erfolgt. Der Transport von Proteinen aus dem Cytoplasma erfolgt nach an sich bekannten Methoden, wie sie beispielsweise in der EP-B 0 006 694 beschrieben sind.

Als Steuerungsregion kommt jede geeignete regulierbare Genregulationsregion in Betracht, beispielsweise lac, tac trp oder synthetische Sequenzen. Besonders bevorzugt sind Regulationsregionen, die zuverlässig abschaltbar sind.

Die Isolierung der Proteine aus dem periplasmatischen Raum erfolgt vorteilhaft dadurch, daß man auf die abgeernteten Zellen einen milden osmotischen Schock ausübt und die hierbei erhaltene flüssige Phase durch Ultrafiltration oder Fällung, beispielsweise mit Salzen wie Ammoniumsulfat, aufkonzentriert.

Ein "milder" osmotischer Schock bewirkt das Austreten des Periplasmas, wobei aber die cytoplasmatische Membran intakt bleibt.

Das Proteinkonzentrat wird zweckmäßig nach einer Dialyse auf ein Adsorbens, vorteilhaft in Form einer Affinitätssäule, gegeben, das mit dem entsprechenden Antigen oder Hapten beladen ist. Durch geeignete Elution, vorteilhaft mit dem Antigen oder Hapten, erhält man dann den Antikörper bzw. das funktionelle Antikörperfragment.

In der eukaryotischen Zelle werden Antikörper im Lumen des endoplasmatischen Retikulums - wahrscheinlich unter Mitwirkung von Disulfid-Isomerasen, Prolin-cis-trans-Isomerasen und möglicherweise weiteren Enzymen oder Proteinen- gebildet. Es war überraschend, daß auch die Bakterienzelle in der Lage ist, die beiden Ketten in der etwa gleichen stöchiometrischen Menge herzustellen, beide Vorläuferproteine in den periplasmatischen Raum oder das sie umgebende Medium zu transportieren, die Signalsequenzen korrekt abzuspalten, die globulären und löslichen Domänen korrekt zu falten, die intramolekularen Disulfidbindungen zu bilden und beide Ketten zu einem Heterodimer zu assoziieren, da es als unwahrscheinlich gilt, daß die Bakterienzelle über solche oder gleichwirkende Enzyme verfügt. Es zeigte sich also überraschenderweise, daß im Falle Gram-negativer Bakterien das bakterielle Periplasma dem Lumen des eukaryotischen endoplasmatischen Retikulums in dieser Beziehung funktionell äquivalent ist.

Das erfindungsgemäße Verfahren hat eine Reihe von Vorteilen: Abgesehen von der schon erwähnten leichten und billigen bakteriellen Massenfermentation werden unmittelbar funktionelle Proteine gebildet, wodurch also die Spaltung von Fusionsproteinen mit nachfolgender Isolierung des gewünschten Proteins oder Protein-Fragments, dessen Oxidation oder in-vitro-Rückfaltung vermieden werden.

Beim erfindungsgemäßen Verfahren wurden auch keine Probleme mit zellulären Proteasen festgestellt. Bekanntlich werden ja wegen dieser zellulären Proteasen Proteine in Bakterien üblicherweise in der Form von Fusionsproteinen, insbesondere als unlösliche Einschlußkörper, exprimiert, was aber die genannten aufwendigen Weiterverarbeitungsschritte mit sich bringt. Demgegenüber ist die Abtrennung und Reinigung bis zur Homogenität beim erfindungsgemäßen Verfahren schnell und einfach.

Die Erfindung erlaubt also einen leichten Zugang zu Antikörpern, ihren funktionellen Fragmenten und abgewandelten Antikörpern, die sich durch Einfügung, Eliminierung und/oder Austausch von Aminosäuren von den nativen Antikörpern unterscheiden. So kann man beispielsweise Cysteine eliminieren oder durch andere Aminosäuren ersetzen, um eine unerwünschte Faltung zu unterdrücken. Genauso kann man einen murinen Antikörper in einen humanen überführen, oder andere Mutationen einführen. Neben den pharmakologischen und technischen Anwendungsmöglichkeiten besteht somit ein erleichteter Zugang zur Erforschung der Antikörperstruktur und -funktion und der Grundlagen der enzymatischen Katalyse (V. Raso and B.D. Stollor, Biochemistry 14, 584 (1975); V. Raso and B.D. Stollar, Biochemistry 14, 591 (1975); A. Tramontano, K.D. Janda and R.A Lerner, Science 234, 1566 (1986); S.J. Pollack, J.W. Jacobs and P.G. Schultz, Science 234, 1570 (1986); J. Jacobs, P.G. Schultz, R. Sugasawara and M. Powell, J. Am. Chem. Soc. 109, 2174 (1987)).

Die Erfindung erlaubt weiterhin die Anwendung von Testsystemen (Assays) unmittelbar auf die bakterielle Zelle, in der der funktionelle Antikörper gebildet wird, und damit eine schnelle Untersuchung auf gegebenenfalls mutierte Antikörper.

Neben den genannten Variationen des Antikörpermoleküls durch Variation einzelner oder weniger Aminosäuren können in das Antikörper-Gen auch andere Genregionen eingefügt oder Teile gegen unkritische Genregionen ausgetauscht werden. Man kann so Markierungsenzyme (M.S. Neuberger, G.T. Williams and R.O. Fox, Nature 312, 604 (1984)), Toxine (G. Möller (ed.) "Antibody carriers of drugs and toxins in tumor therapy", Immunol. Rev. 62, Munksgaard, Copenhagen (1982)) oder Immunglobulin-Regionen einer anderen Klasse (M.S. Neuberger, G.T. Williams, E.B. Mitchell, S.S. Jouhal, J.G. Flanagan, and T.H. Rabbitts, Nature 314, 268 (1985)) oder einer anderen Species (P.T. Jones, P.H. Dear, J. Foote, M.S. Neuberger, and G. Winter, Nature 321, 522 (1986)) an das Antikörpermolekül koppeln.

Das erfindungsgemäße Verfahren wird im folgenden am Beispiel der variablen Domänen des Phosphorylcholin-bindenden Antikörper-Myelom-Proteins McPC603 erläutert. Die dreidimensionale Struktur dieses Mäuse-Immunglobulin A ist bekannt (D.M. Segal, E. A. Padlan, G.H. Cohen, S. Rudikoff, M. Potter and D.R. Davies, Proc. Natl. Acad. Sci. 71, 4298 (1974)). Eingesetzt wurden synthetische Gene für die variable leichte Kette $V_L$ und schwere Kette $V_H$. Solche synthetischen Gene sind in der deutschen Offenlegungsschrift 37 15 033 bzw. der EP-A 0 290 005 und der AU-A 15631/88 (dort Tabellen 1 und 2) vorgeschlagen. Eine besonders zweckmäßige Ausgestaltung solcher synthetischen Gene zeigt die Tabelle, in der die DNA-Sequenz des gesamten Expressionsplasmids wiedergegeben ist und die Gene für die beiden Ketten durch die Aminosäurenangabe hervorgehoben sind. Bei der Konstruktion dieser DNA-Sequenzen wurden Kodons vermieden, die von E. coli selten verwendet werden. Weiterhin wurden singuläre Restriktionsenzym-Schnittstellen eingebaut und auf die Sekundärstruktur der RNA Rücksicht genommen.

Bei dem als Modell dienenden funktionellen Antikörper-Fragment hat jede Domäne eine intramolekulare Disulfidbrücke (von Cys 23 zu Cys 94 in $V_L$ und von Cys 22 zu Cys 98 in $V_H$). Es existiert keine Disulfidbrücke zwischen den Ketten und auch keine freies Cystein.

Der eingesetzte Expressionsvektor pASK 22 ist schematisch in der folgenden Formel

wiedergegeben. In ihr sind die synthetischen Gene für die $V_L$- und die $V_H$-Domänen an Genfragmente für die bakterielle Signalsequenz des Äußeren Membranproteins A (ompA) einerseits und der Alkalischen Phosphatase (phoA) andererseits gekoppelt. Die Gene für die beiden Vorläuferproteine sind in einer künstlichen Operon-artigen Struktur hinter dem lac-Promotor angeordnet, wodurch die gleichzeitige Induktion, Koexpression und Kosekretion beider Gene gewährleistet sind.

Nach der Induktion der Genexpression werden die Zellen geerntet und einem milden osmotischen Schock ausgesetzt. Die hierbei erhaltene Flüssigphase, die die periplasmatischen Proteine enthält, wird durch Ultrafiltration konzentriert, dialysiert und direkt auf eine Affinitätssäule aufgetragen, die ein Phosphorylcholinderivat (B. Chesebro and H. Metzger, Biochemistry 11, 766 (1972)) als Affinitätsliganden enthält. Durch Elution mit Phosphorylcholin erhält man ein homogenes $F_V$-Fragment, das gelelektrophoretisch einheitlich ist. Aus dem SDS-Polyacrylamidgel läßt sich ableiten, daß beide Ketten des gereinigten $F_V$-Fragments in einem Molverhältnis von 1:1 vorhanden sind und die erwarteten Molgewichte der reifen Proteine ($V_H$:13600 D, $V_L$: 12400 D) vorliegen. Zum Nachweis der korrekten Abspaltung beider Signalsequenzen wurden die sechs N-terminalen Aminosäuren beider Ketten sequenziert. Es ergab sich, daß beide Ketten die korrekten N-Termini für die reifen Proteine aufwiesen. Es wurden also beide heterologen Präproteine durch die bakterielle Signalpeptidase richtig gespalten und es ist kein Anhaltspunkt erkennbar, daß eine unpräzise Prozessierung oder eine N-terminale Abbaureaktion erfolgte.

Die Affinitätskonstante des rekombinanten $F_V$-Fragments von McPC603 wurde durch Gleichgewichtsdialyse gemessen. Hierbei wurden die gleichen Bedingungen verwendet, die bei der Bestimmung der Affinitätskonstante des nativen Antikörpers McP603, isoliert aus Maus-Aszites, angewandt wurden. Der für das $F_V$-Fragment gefundene Wert von $1,21 \pm 0,06 \times 10^5 M^{-1}$ ist innerhalb der Experimentiergenauigkeit identisch mit dem für den nativen Antikörper berichteten Wert $1,6 \pm 0,4 \times 10^5 M^{-1}$. Die Bindungskurve nach Scatchard (Ann. N. Y. Acad. Sci. 51, 660 (1949)) ist linear und die Extrapolation zeigt an, daß annähernd 1 Mol Hapten pro Mol $F_V$-Fragment gebunden sind. Hierdurch wird bestätigt, daß es nur eine Art von Bindungsstellen pro $F_V$-Fragment gibt und daß in dem isolierten Protein keine inaktiven Bestandteile vorhanden sind.

Es hat sich also überraschenderweise gezeigt, daß man das $F_V$-Fragment des Antikörpers McPC603 als völlig funktionelles und stabiles Protein in E. coli herstellen kann. Hierdurch wird die funktionelle Äquivalenz des Transports in das Periplasma der Bakterienzelle zum Transport in das Lumen des endoplasmatischen Retikulums der eukaryotischen Zelle bewiesen. Diese Äquivalenz ist unbekannt und auch unvermutet, da das bisher am besten charakterisierte bakterielle Protein, das als lösliches heterodimeres Protein im Periplasma definiert werden könnte, die E. coli-Penicillin-Acylase, durch proteolytische Prozessierung aus einem einzelkettigen Vorläufer im Periplasma entsteht (G. Schumacher, D. Sizmann, H. Hang, P. Buckel and A. Böck, Nucleic Acids Res. 14, 5713 (1986)). Außerdem wurde bereits darauf hingewiesen, daß nach derzeitiger Kenntnis Bakterien keine Enzyme oder Proteine besitzen, die in Eukaryonten an der Faltung beteiligt sein könnten.

Überraschend war auch, daß das $F_V$-Fragment von McPC603 im wesentlichen die gleiche Affinitätskonstante für Phosphorylcholin hat wie der intakte Antikörper McPC603 selbst. Dieser Befund ist unerwartet, da die Funktionalität von $F_V$-Fragmenten in der Literatur umstritten ist (J. Sen and S. Beychok, Proteins 1, 256 (1986)). Hierdurch zeigt sich, daß eine Modifikation oder sogar komplette Deletion der konstanten Domänen die Funktionalität erhalten kann.

Im Gegensatz zu der bisher einzigen Methode zur Herstellung von $F_V$-Fragmenten, nämlich durch Proteolyse eines Antikörpers, ergeben sich beim erfindungsgemäßen Verfahren keine Probleme mit nicht-funktionellen, nicht korrekt gefalteten, nicht korrekt reassoziierten oder chemisch modifizierten Proteinen. Im übrigen eignet sich das bevorzugte Isolierungsverfahren mit Hilfe eines Antigen-oder Hapten-beladenen Adsorbens auch dazu, solche nach bekannten Verfahren erhaltenen $F_V$-Fragmente zu reinigen, da solche Verunreinigungen entweder nicht gebunden oder nicht eluiert würden.

### Beispiel 1: Herstellung des Plasmids pASK22:

Das Expressionsplasmid pASK22 wird aus dem großen EcoRI-HindIII-Fragment von pUC12 (C. Yanisch-Perron, J. Vieira, and J. Messing, Gene 33, 103 (1985)), aus Fragmenten der Vektoren pIN III-OmpA1 (Y. Masui, J. Coleman and M. Inouye in "Experimental manipulation of gene expression", M. Inouye, ed., Academic Press 1983) und pHI61 (H. Inouye, W. Barnes and J. Beckwith, J. Bacteriol. 149, 434 (1982)) sowie verschiedenen synthetischen DNA-Fragmenten in mehreren Stufen mit Hilfe von an sich bekannten Methoden (T. Maniatis, E. F. Fritsch and J. Sambrook, Molecular Cloning, Cold Spring Harbor 1982) konstruiert. Die vollständige DNA-Sequenz des so erhaltenen Vektors pASK22 ist in der Tabelle dargestellt.

Mit dem Ligationsgemisch werden kompetente E. coli-Zellen transformiert und auf Ampicillinresistenz selektiert. Die Plasmide mit der gewünschten Genstruktur werden durch Restriktionsanalyse sowie Sequenzierung der kritischen Übergänge charakterisiert.

### Beispiel 2: Herstellung des $F_V$-Fragments

Eine Kultur des mit pASK22 transformierten E. coli-Stammes W3110 wird in LB-Medium mit 100 mg/l Ampicillin bis zu einer $OD_{550}$ von 0,5 gezüchtet. Durch Zugabe von IPTG bis zu einer Endkonzentration von 1mM wird die Expression induziert. Nach 45 Minuten werden die Zellen durch Zentrifugieren bei 4000 g (10 Minuten bei 4°C) geerntet. Durch Resuspendieren des Zellpellets in TES-Puffer (0,2 M Tris•HCl, pH 8,0; 0,5 mM EDTA; 0,5 M Saccharose) in 10 ml/l der ursprünglichen Kultur wird eine Zellfraktionierung vorgenommen. Die Zellen werden durch Zugabe von 15 ml/l der ursprünglichen Kultur TES-Puffer, der 1:4 mit Wasser verdünnt ist und 2 mM Phosphorylcholin enthält, einem milden osmotischen Schock ausgesetzt. Nach 30 Minuten Inkubation auf Eis wird die Suspension 10 Minuten bei 5000 g zentrifugiert und der Überstand erneut 15 Minuten bei 48 000 g zentrifugiert. Der so erhaltene Überstand, der alle löslichen periplasmatischen Proteine enthält, wird durch Ultrafiltration ([R]AMICON YM5 Membran) auf ein Volumen von etwa 2,5 ml/l der ursprünglichen Kultur konzentriert und gegen BBS-Puffer (0,2 M Borat/NaOH, pH 8,0; 0,16 M NaCl) dialysiert. Diese konzentrierte Lösung wird auf eine mit einem Phosphorylcholin-Derivat (B. Chesebro and H. Metzger, a. a. O.) beladene Affinitätssäule gegeben (1-4 ml Lösung pro ml Bettvolumen), mit BBS-Puffer gewaschen und das reine $F_V$-Fragment mit einer Lösung von 1 mM Phosphorylcholin in BBS-Puffer eluiert.

### Beispiel 3: Gleichgewichtsdialyse

In eine Dialysierkammer mit jeweils 100 $\mu$l Volumen auf jeder Seite der Membran wurden auf eine Seite 50 $\mu$l gereinigtes $F_V$-Fragment in BBS-Puffer und auf die andere Seite eine Lösung von 50 $\mu$l Phosphoryl-(methyl-$^{14}$C) Cholin (50 mCi/mMol) in BBS-Puffer gefüllt. Aus der $OD_{205}$ von 6,85 wurde für das $F_V$-Fragment eine Konzentration von 0,22 mg/ml ermittelt (R.K. Scopes, Protein Purification-Principles and Practice, Springer-Verlag, New York, 1982, S. 241). Nach einer Gleichgewichtseinstellung von 22 Stunden bei Raumtemperatur wurden 20 $\mu$l-Proben jeder Lösung in einem Szintillationszähler (Beckman LS 1801) gemessen und die Daten nach Scatchard (a.a.O.) aufgetragen. Aus der Steigung der erhaltenen Linie ergibt sich eine Affinitätskonstante von $K_a = 1,21 \pm 0,06 \times 10^5$ $M^{-1}$.

TABELLE

DNA-Sequenz von pASK22 mit Aminosäure-Sequenzen
von ompA-V$_H$ und phoA-V$_L$

```
     GCGCCCAATACGCAAACCGCCTCTCCCCGCGCGTTGGCCGATTCATTAATGCAGCTGGCA
  1  ---------+---------+---------+---------+---------+---------+ 60
     CGCGGGTTATGCGTTTGGCGGAGAGGGGCGCGCAACCGGCTAAGTAATTACGTCGACCGT


     CGACAGGTTTCCCGACTGGAAAGCGGGCAGTGAGCGCAACGCAATTAATGTGAGTTAGCT
 61  ---------+---------+---------+---------+---------+---------+ 120
     GCTGTCCAAAGGGCTGACCTTTCGCCCGTCACTCGCGTTGCGTTAATTACACTCAATCGA


     CACTCATTAGGCACCCCAGGCTTTACACTTTATGCTTCCGGCTCGTATGTTGTGTGGAAT
121  ---------+---------+---------+---------+---------+---------+ 180
     GTGAGTAATCCGTGGGGTCCGAAATGTGAAATACGAAGGCCGAGCATACAACACACCTTA


     TGTGAGCGGATAACAATTTCACACAGGAAACAGCTATGACCATGATTACGAATTTCTAGA
181  ---------+---------+---------+---------+---------+---------+ 240
     ACACTCGCCTATTGTTAAAGTGTGTCCTTTGTCGATACTGGTACTAATGCTTAAAGATCT


     TAACGAGGGCAAAAAAATGAAAAAGACAGCTATCGCGATTGCAGTGGCACTGGCTGGTTTC
241  ---------+---------+---------+---------+---------+---------+ 300
     ATTGCTCCCGTTTTTTTACTTTTTCTGTCGATAGCGCTAACGTCACCGTGACCGACCAAAG

                         MetLysLysThrAlaIleAlaIleAlaValAlaLeuAlaGlyPhe -

     GCTACCGTAGCGCAGGCCGAAGTTAAACTGGTAGAGTCTGGTGGTGGTCTGGTACAGCCG
301  ---------+---------+---------+---------+---------+---------+ 360
     CGATGGCATCGCGTCCGGCTTCAATTTGACCATCTCAGACCACCACCAGACCATGTCGGC

     AlaThrValAlaGlnAlaGluValLysLeuValGluSerGlyGlyGlyLeuValGlnPro -

     GGTGGATCCCTGCGTCTGTCTTGCGCTACCTCAGGTTTCACCTTCTCTGACTTCTACATG
361  ---------+---------+---------+---------+---------+---------+ 420
     CCACCTAGGGACGCAGACAGAACGCGATGGAGTCCAAAGTGGAAGAGACTGAAGATGTAC

     GlyGlySerLeuArgLeuSerCysAlaThrSerGlyPheThrPheSerAspPheTyrMet -

     GAGTGGGTACGTCAGCCCCCGGGTAAACGTCTCGAGTGGATCGCAGCTAGCCGTAACAAA
421  ---------+---------+---------+---------+---------+---------+ 480
     CTCACCCATGCAGTCGGGGGCCCATTTGCAGAGCTCACCTAGCGTCGATCGGCATTGTTT

     GluTrpValArgGlnProProGlyLysArgLeuGluTrpIleAlaAlaSerArgAsnLys -

     GGTAACAAGTATACCACCGAATACAGCGCTTCTGTTAAAGGTCGTTTCATCGTTTCTCGT
481  ---------+---------+---------+---------+---------+---------+ 540
     CCATTGTTCATATGGTGGCTTATGTCGCGAAGACAATTTCCAGCAAAGTAGCAAAGAGCA

     GlyAsnLysTyrThrThrGluTyrSerAlaSerValLysGlyArgPheIleValSerArg -

     GACACTAGTCAATCGATCCTGTACCTGCAGATGAATGCATTGCGTGCTGAAGACACCGCT
541  ---------+---------+---------+---------+---------+---------+ 600
     CTGTGATCAGTTAGCTAGGACATGGACGTCTACTTACGTAACGCACGACTTCTGTGGCGA

     AspThrSerGlnSerIleLeuTyrLeuGlnMetAsnAlaLeuArgAlaGluAspThrAla -
```

```
       ATCTACTACTGCGCGCGTAACTACTATGGCAGCACTTGGTACTTCGACGTTTGGGGTGCA
601    ---------+---------+---------+---------+---------+---------+  660
       TAGATGATGACGCGCGCATTGATGATACCGTCGTGAACCATGAAGCTGCAAACCCCACGT

       IleTyrTyrCysAlaArgAsnTyrTyrGlySerThrTrpTyrPheAspValTrpGlyAla  -

       GGTACCACCGTTACCGTTTCTTCTTGATAACATGGAGAAAATAAAGTGAAACAAAGCACT
661    ---------+---------+---------+---------+---------+---------+  720
       CCATGGTGGCAATGGCAAAGAAGAACTATTGTACCTCTTTTATTTCACTTTGTTTCGTGA

       GlyThrThrValThrValSerSerEnd                      MetLysGlnSerThr  -

       ATTGCACTGGCACTCTTACCGTTACTGTTTACCCCTGTGACAAAAGCCGATATCGTTATG
721    ---------+---------+---------+---------+---------+---------+  780
       TAACGTGACCGTGAGAATGGCAATGACAAATGGGGACACTGTTTTCGGCTATAGCAATAC

       IleAlaLeuAlaLeuLeuProLeuLeuPheThrProValThrLysAlaAspIleValMet  -

       ACCCAGTCTCCGAGCTCTCTGTCTGTATCTGCAGGTGAACGTGTTACCATGTCTTGCAAA
781    ---------+---------+---------+---------+---------+---------+  840
       TGGGTCAGAGGCTCGAGAGACAGACATAGACGTCCACTTGCACAATGGTACAGAACGTTT

       ThrGlnSerProSerSerLeuSerValSerAlaGlyGluArgValThrMetSerCysLys  -

       TCTTCTCAGTCTCTGCTGAACTCTGGTAACCAGAAAAACTTCCTGGCGTGGTATCAGCAA
841    ---------+---------+---------+---------+---------+---------+  900
       AGAAGAGTCAGAGACGACTTGAGACCATTGGTCTTTTTGAAGGACCGCACCATAGTCGTT

       SerSerGlnSerLeuLeuAsnSerGlyAsnGlnLysAsnPheLeuAlaTrpTyrGlnGln  -

       AAGCCTGGCCAACCGCCGAAACTGCTGATCTACGGTGCGTCGACCCGTGAATCTGGTGTT
901    ---------+---------+---------+---------+---------+---------+  960
       TTCGGACCGGTTGGCGGCTTTGACGACTAGATGCCACGCAGCTGGGCACTTAGACCACAA

       LysProGlyGlnProProLysLeuLeuIleTyrGlyAlaSerThrArgGluSerGlyVal  -

       CCGGACCGTTTTACCGGTAGCGGTAGCGGTACCGACTTCACTCTGACCATCTCTTCTGTA
961    ---------+---------+---------+---------+---------+---------+  1020
       GGCCTGGCAAAATGGCCATCGCCATCGCCATGGCTGAAGTGAGACTGGTAGAGAAGACAT

       ProAspArgPheThrGlySerGlySerGlyThrAspPheThrLeuThrIleSerSerVal  -

       CAGGCTGAAGATCTGGCTGTTTACTACTGTCAAAACGACCACTCTTACCCGCTGACCTTT
1021   ---------+---------+---------+---------+---------+---------+  1080
       GTCCGACTTCTAGACCGACAAATGATGACAGTTTTGCTGGTGAGAATGGGCGACTGGAAA

       GlnAlaGluAspLeuAlaValTyrTyrCysGlnAsnAspHisSerTyrProLeuThrPhe  -

       GGCGCCGGCACCAAACTGGAACTGAAGCGCGCTTGATAAGCTTGGCACTGGCCGTCGTTT
1081   ---------+---------+---------+---------+---------+---------+  1140
       CCGCGGCCGTGGTTTGACCTTGACTTCGCGCGAACTATTCGAACCGTGACCGGCAGCAAA

       GlyAlaGlyThrLysLeuGluLeuLysArgAlaEnd

       TACAACGTCGTGACTGGGAAAACCCTGGCGTTACCCAACTTAATCGCCTTGCAGCACATC
1141   ---------+---------+---------+---------+---------+---------+  1200
       ATGTTGCAGCACTGACCCTTTTGGGACCGCAATGGGTTGAATTAGCGGAACGTCGTGTAG

       CCCCTTTCGCCAGCTGGCGTAATAGCGAAGAGGCCCGCACCGATCGCCCTTCCCAACAGT
1201   ---------+---------+---------+---------+---------+---------+  1260
       GGGGAAAGCGGTCGACCGCATTATCGCTTCTCCGGGCGTGGCTAGCGGGAAGGGTTGTCA
```

```
       TGCGCAGCCTGAATGGCGAATGGCGCCTGATGCGGTATTTTCTCCTTACGCATCTGTGCG
1261   ---------+---------+---------+---------+---------+---------+ 1320
       ACGCGTCGGACTTACCGCTTACCGCGGACTACGCCATAAAAGAGGAATGCGTAGACACGC

       GTATTTCACACCGCATATGGTGCACTCTCAGTACAATCTGCTCTGATGCCGCATAGTTAA
1321   ---------+---------+---------+---------+---------+---------+ 1380
       CATAAAGTGTGGCGTATACCACGTGAGAGTCATGTTAGACGAGACTACGGCGTATCAATT

       GCCAGCCCCGACACCCGCCAACACCCGCTGACGCGCCCTGACGGGCTTGTCTGCTCCCGG
1381   ---------+---------+---------+---------+---------+---------+ 1440
       CGGTCGGGGCTGTGGGCGGTTGTGGGCGACTGCGCGGGACTGCCCGAACAGACGAGGGCC

       CATCCGCTTACAGACAAGCTGTGACCGTCTCCGGGAGCTGCATGTGTCAGAGGTTTTCAC
1441   ---------+---------+---------+---------+---------+---------+ 1500
       GTAGGCGAATGTCTGTTCGACACTGGCAGAGGCCCTCGACGTACACAGTCTCCAAAAGTG

       CGTCATCACCGAAACGCGCGAGACGAAAGGGCCTCGTGATACGCCTATTTTTATAGGTTA
1501   ---------+---------+---------+---------+---------+---------+ 1560
       GCAGTAGTGGCTTTGCGCGCTCTGCTTTCCCGGAGCACTATGCGGATAAAAATATCCAAT

       ATGTCATGATAATAATGGTTTCTTAGACGTCAGGTGGCACTTTTCGGGGAAATGTGCGCG
1561   ---------+---------+---------+---------+---------+---------+ 1620
       TACAGTACTATTATTACCAAAGAATCTGCAGTCCACCGTGAAAAGCCCCTTTACACGCGC

       GAACCCCTATTTGTTTATTTTTCTAAATACATTCAAATATGTATCCGCTCATGAGACAAT
1621   ---------+---------+---------+---------+---------+---------+ 1680
       CTTGGGGATAAACAAATAAAAAGATTTATGTAAGTTTATACATAGGCGAGTACTCTGTTA

       AACCCTGATAAATGCTTCAATAATATTGAAAAAGGAAGAGTATGAGTATTCAACATTTCC
1681   ---------+---------+---------+---------+---------+---------+ 1740
       TTGGGACTATTTACGAAGTTATTATAACTTTTTCCTTCTCATACTCATAAGTTGTAAAGG

       GTGTCGCCCTTATTCCCTTTTTTGCGGCATTTTGCCTTCCTGTTTTTGCTCACCCAGAAA
1741   ---------+---------+---------+---------+---------+---------+ 1800
       CACAGCGGGAATAAGGGAAAAAACGCCGTAAAACGGAAGGACAAAAACGAGTGGGTCTTT

       CGCTGGTGAAAGTAAAAGATGCTGAAGATCAGTTGGGTGCACGAGTGGGTTACATCGAAC
1801   ---------+---------+---------+---------+---------+---------+ 1860
       GCGACCACTTTCATTTTCTACGACTTCTAGTCAACCCACGTGCTCACCCAATGTAGCTTG

       TGGATCTCAACAGCGGTAAGATCCTTGAGAGTTTTCGCCCCGAAGAACGTTTTCCAATGA
1861   ---------+---------+---------+---------+---------+---------+ 1920
       ACCTAGAGTTGTCGCCATTCTAGGAACTCTCAAAAGCGGGGCTTCTTGCAAAAGGTTACT

       TGAGCACTTTTAAAGTTCTGCTATGTGGCGCGGTATTATCCCGTATTGACGCCGGGCAAG
1921   ---------+---------+---------+---------+---------+---------+ 1980
       ACTCGTGAAAATTTCAAGACGATACACCGCGCCATAATAGGGCATAACTGCGGCCCGTTC

       AGCAACTCGGTCGCCGCATACACTATTCTCAGAATGACTTGGTTGAGTACTCACCAGTCA
1981   ---------+---------+---------+---------+---------+---------+ 2040
       TCGTTGAGCCAGCGGCGTATGTGATAAGAGTCTTACTGAACCAACTCATGAGTGGTCAGT
```

9

```
      CAGAAAAGCATCTTACGGATGGCATGACAGTAAGAGAATTATGCAGTGCTGCCATAACCA
2041  ---------+---------+---------+---------+---------+---------+ 2100
      GTCTTTTCGTAGAATGCCTACCGTACTGTCATTCTCTTAATACGTCACGACGGTATTGGT
```

```
      TGAGTGATAACACTGCGGCCAACTTACTTCTGACAACGATCGGAGGACCGAAGGAGCTAA
2101  ---------+---------+---------+---------+---------+---------+ 2160
      ACTCACTATTGTGACGCCGGTTGAATGAAGACTGTTGCTAGCCTCCTGGCTTCCTCGATT
```

```
      CCGCTTTTTTGCACAACATGGGGGATCATGTAACTCGCCTTGATCGTTGGGAACCGGAGC
2161  ---------+---------+---------+---------+---------+---------+ 2220
      GGCGAAAAAACGTGTTGTACCCCCTAGTACATTGAGCGGAACTAGCAACCCTTGGCCTCG
```

```
      TGAATGAAGCCATACCAAACGACGAGCGTGACACCACGATGCCTGTAGCAATGGCAACAA
2221  ---------+---------+---------+---------+---------+---------+ 2280
      ACTTACTTCGGTATGGTTTGCTGCTCGCACTGTGGTGCTACGGACATCGTTACCGTTGTT
```

```
      CGTTGCGCAAACTATTAACTGGCGAACTACTTACTCTAGCTTCCCGGCAACAATTAATAG
2281  ---------+---------+---------+---------+---------+---------+ 2340
      GCAACGCGTTTGATAATTGACCGCTTGATGAATGAGATCGAAGGGCCGTTGTTAATTATC
```

```
      ACTGGATGGAGGCGGATAAAGTTGCAGGACCACTTCTGCGCTCGGCCCTTCCGGCTGGCT
2341  ---------+---------+---------+---------+---------+---------+ 2400
      TGACCTACCTCCGCCTATTTCAACGTCCTGGTGAAGACGCGAGCCGGGAAGGCCGACCGA
```

```
      GGTTTATTGCTGATAAATCTGGAGCCGGTGAGCGTGGGTCTCGCGGTATCATTGCAGCAC
2401  ---------+---------+---------+---------+---------+---------+ 2460
      CCAAATAACGACTATTTAGACCTCGGCCACTCGCACCCAGAGCGCCATAGTAACGTCGTG
```

```
      TGGGGCCAGATGGTAAGCCCTCCCGTATCGTAGTTATCTACACGACGGGGAGTCAGGCAA
2461  ---------+---------+---------+---------+---------+---------+ 2520
      ACCCCGGTCTACCATTCGGGAGGGCATAGCATCAATAGATGTGCTGCCCCTCAGTCCGTT
```

```
      CTATGGATGAACGAAATAGACAGATCGCTGAGATAGGTGCCTCACTGATTAAGCATTGGT
2521  ---------+---------+---------+---------+---------+---------+ 2580
      GATACCTACTTGCTTTATCTGTCTAGCGACTCTATCCACGGAGTGACTAATTCGTAACCA
```

```
      AACTGTCAGACCAAGTTTACTCATATATACTTTAGATTGATTTAAAACTTCATTTTTAAT
2581  ---------+---------+---------+---------+---------+---------+ 2640
      TTGACAGTCTGGTTCAAATGAGTATATATGAAATCTAACTAAATTTTGAAGTAAAAATTA
```

```
      TTAAAAGGATCTAGGTGAAGATCCTTTTTGATAATCTCATGACCAAAATCCCTTAACGTG
2641  ---------+---------+---------+---------+---------+---------+ 2700
      AATTTTCCTAGATCCACTTCTAGGAAAAACTATTAGAGTACTGGTTTTAGGGAATTGCAC
```

```
      AGTTTTCGTTCCACTGAGCGTCAGACCCCGTAGAAAAGATCAAAGGATCTTCTTGAGATC
2701  ---------+---------+---------+---------+---------+---------+ 2760
      TCAAAAGCAAGGTGACTCGCAGTCTGGGGCATCTTTTCTAGTTTCCTAGAAGAACTCTAG
```

```
      CTTTTTTTCTGCGCGTAATCTGCTGCTTGCAAACAAAAAAACCACCGCTACCAGCGGTGG
2761  ---------+---------+---------+---------+---------+---------+ 2820
      GAAAAAAAGACGCGCATTAGACGACGAACGTTTGTTTTTTTGGTGGCGATGGTCGCCACC
```

10

```
      TTTGTTTGCCGGATCAAGAGCTACCAACTCTTTTTCCGAAGGTAACTGGCTTCAGCAGAG
2821  ---------+---------+---------+---------+---------+---------+ 2880
      AAACAAACGGCCTAGTTCTCGATGGTTGAGAAAAAGGCTTCCATTGACCGAAGTCGTCTC


      CGCAGATACCAAATACTGTCCTTCTAGTGTAGCCGTAGTTAGGCCACCACTTCAAGAACT
2881  ---------+---------+---------+---------+---------+---------+ 2940
      GCGTCTATGGTTTATGACAGGAAGATCACATCGGCATCAATCCGGTGGTGAAGTTCTTGA


      CTGTAGCACCGCCTACATACCTCGCTCTGCTAATCCTGTTACCAGTGGCTGCTGCCAGTG
2941  ---------+---------+---------+---------+---------+---------+ 3000
      GACATCGTGGCGGATGTATGGAGCGAGACGATTAGGACAATGGTCACCGACGACGGTCAC


      GCGATAAGTCGTGTCTTACCGGGTTGGACTCAAGACGATAGTTACCGGATAAGGCGCAGC
3001  ---------+---------+---------+---------+---------+---------+ 3060
      CGCTATTCAGCACAGAATGGCCCAACCTGAGTTCTGCTATCAATGGCCTATTCCGCGTCG


      GGTCGGGCTGAACGGGGGGTTCGTGCACACAGCCCAGCTTGGAGCGAACGACCTACACCG
3061  ---------+---------+---------+---------+---------+---------+ 3120
      CCAGCCCGACTTGCCCCCCAAGCACGTGTGTCGGGTCGAACCTCGCTTGCTGGATGTGGC


      AACTGAGATACCTACAGCGTGAGCTATGAGAAAGCGCCACGCTTCCCGAAGGGAGAAAGG
3121  ---------+---------+---------+---------+---------+---------+ 3180
      TTGACTCTATGGATGTCGCACTCGATACTCTTTCGCGGTGCGAAGGGCTTCCCTCTTTCC


      CGGACAGGTATCCGGTAAGCGGCAGGGTCGGAACAGGAGAGCGCACGAGGGAGCTTCCAG
3181  ---------+---------+---------+---------+---------+---------+ 3240
      GCCTGTCCATAGGCCATTCGCCGTCCCAGCCTTGTCCTCTCGCGTGCTCCCTCGAAGGTC


      GGGGAAACGCCTGGTATCTTTATAGTCCTGTCGGGTTTCGCCACCTCTGACTTGAGCGTC
3241  ---------+---------+---------+---------+---------+---------+ 3300
      CCCCTTTGCGGACCATAGAAATATCAGGACAGCCCAAAGCGGTGGAGACTGAACTCGCAG


      GATTTTTGTGATGCTCGTCAGGGGGGCGGAGCCTATGGAAAAACGCCAGCAACGCGGCCT
3301  ---------+---------+---------+---------+---------+---------+ 3360
      CTAAAAACACTACGAGCAGTCCCCCCGCCTCGGATACCTTTTTGCGGTCGTTGCGCCGGA


      TTTTACGGTTCCTGGCCTTTTGCTGGCCTTTTGCTCACATGTTCTTTCCTGCGTTATCCC
3361  ---------+---------+---------+---------+---------+---------+ 3420
      AAAATGCCAAGGACCGGAAAACGACCGGAAAACGAGTGTACAAGAAAGGACGCAATAGGG


      CTGATTCTGTGGATAACCGTATTACCGCCTTTGAGTGAGCTGATACCGCTCGCCGCAGCC
3421  ---------+---------+---------+---------+---------+---------+ 3480
      GACTAAGACACCTATTGGCATAATGGCGGAAACTCACTCGACTATGGCGAGCGGCGTCGG


      GAACGACCGAGCGCAGCGAGTCAGTGAGCGAGGAAGCGGAAGA
3481  ---------+---------+---------+---------+--- 3523
      CTTGCTGGCTCGCGTCGCTCAGTCACTCGCTCCTTCGCCTTCT
```

## Patentansprüche

1. Verfahren zur Herstellung von funktionellen Antikörpern, funktionellen Fragmenten von Antikörpern oder Fusionsproteinen aus funktionellen Antikörperdomänen und anderen Proteinen dadurch gekennzeichnet, daß man die Gene für die Schwere und leichte Kette an je eine Signalsequenz koppelt, die den Transport der Polypeptidketten durch die cytoplasmatische Membran einer Bakterienzelle bewirken und dann abgespalten werden, die Genstrukturen in einem Bakterium zur Expression bringt und das funktionelle Protein aus dem periplasmatischen Raum oder dem Medium, isoliert.

2. Verfahren nach Anspruch 1, dadurch gekennzeichnet, daß das Bakterium ein Gram-negatives Bakterium ist.

3. Verfahren nach Anspruch 2, dadurch gekennzeichnet, daß das Bakterium E. coli ist.

4. Verfahren nach Anspruch 1, 2 oder 3, dadurch gekennzeichnet, daß man die Gene für die beiden Präpeptide in der Form eines regulierbaren Operon-Systems koppelt.

5. Verfahren nach einem oder mehreren der vorhergehenden Ansprüche, dadurch gekennzeichnet, daß man zur Isolierung des funktionellen Proteins aus dem periplasmatischen Raum die abgetrennten Bakterien einem solchen osmotischen Schock aussetzt, daß das Periplasma austritt, das Cytoplasma aber intakt bleibt, die hierbei erhaltene flüssige Phase durch Zentrifugieren anreichert und aus diesem Konzentrat die gewünschten Proteine gewinnt.

6. Verfahren nach einem oder mehreren der vorhergehenden Ansprüche, dadurch gekennzeichnet, daß man die das funktionelle Protein enthaltende Lösung auf ein mit dem entsprechenden Antigen oder Hapten beladenes Adsorbens gibt und die gewünschten Proteine durch Elution mit einer dieses Antigen oder Hapten enthaltenden Lösung eluiert.

**Claims**

1. A process for the preparation of functional anti-bodies, functional fragments of antibodies or fusion proteins composed of functional antibody domains and other proteins, which comprises the genes for the heavy and light chain each being coupled to one signal sequence which brings about the transport of the polypeptide chains through the cytoplasmic membrane of a bacterial cell and is then eliminated, bringing about the expression of the gene structures in a bacterium, and isolating the functional protein from the periplasmic space or the medium.

2. The process as claimed in claim 1, wherein the bacterium is a Gram-negative bacterium.

3. The process as claimed in claim 2, wherein the bacterium is E. coli.

4. The process as claimed in claim 1, 2 or 3, wherein the genes for the two prepeptides are coupled in the form of a regulatable operon system.

5. The process as claimed in one or more of the preceding claims, wherein, in order to isolate the functional protein from the periplasmic space, the bacteria which have been separated off are exposed to an osmotic shock such that the periplasm is ejected but the cytoplasm remains intact, the liquid phase obtained by this is enriched by centrifugation, and the desired proteins are obtained from this concentrate.

6. The process as claimed in one or more of the preceding claims, wherein the solution containing the functional protein is applied to an adsorbent loaded with the appropriate antigen or hapten, and the desired proteins are isolated by elution with a solution containing this antigen or hapten.

**Revendications**

1. Procédé pour préparer des anticorps fonctionnels, des fragments fonctionnels d'anticorps ou des protéines de fusion constituées de domaines d'anticorps fonctionnels et d'autres protéines, caractérisé en ce que l'on couple les gènes codant pour la chaîne lourde et la chaîne légère à chacune des séquences-signal, lesquelles provoquent le transport des chaînes polypeptidiques à travers la membrane cytoplasmatique d'une cellule bactérienne et sont ensuite séparées, on exprime les structures des gènes dans une bactérie et on isole la protéine fonctionnelle dans l'espace périplasmatique ou dans le milieu.

2.  Procédé selon la revendication 1, caractérisé en ce que la bactérie est une bactérie à gram négatif.

3.  Procédé selon la revendication 2, caractérisé en ce que la bactérie est E. coli.

4.  Procédé selon la revendication 1, 2 ou 3, caractérisé en ce que l'on couple les gènes codant pour les deux pré-peptides sous la forme d'un système d'opéron régulable.

5.  Procédé selon une ou plusieurs des revendications précédentes, caractérisé en ce que, pour isoler la protéine fonctionnelle de l'espace périplasmatique, on expose les bactéries séparées à un choc osmotique tel que le périplasme sort mais le cytoplasme reste intact, on enrichit la phase liquide ainsi obtenue par centrifugation et on récupère la protéine voulue à partir de ce concentré.

6.  Procédé selon une ou plusieurs des revendications précédentes, caractérisé en ce que l'on verse la solution contenant la protéine fonctionnelle sur un adsorbant chargé avec l'antigène ou le haptène correspondant, et on élue la protéine voulue par élution avec une solution contenant l'un de ces antigène ou haptène.